# EUROPÄISCHE PATENTSCHRIFT

(11) **EP 1 539 921 B1**
(45) Veröffentlichungstag und Bekanntmachung des Hinweises auf die Patenterteilung: **21.11.2007**
(21) Anmeldenummer: 02779357.9
(22) Anmeldetag: 16.09.2002
(51) Int. Cl.: C12M 3/00

(54) **EINRICHTUNG ZUR KULTIVIERUNG VON ZELLEN, INSBESONDERE MENSCHLICHER ODER TIERISCHER ZELLEN**
DEVICE FOR CULTURING CELLS, PARTICULARLY HUMAN OR ANIMAL CELLS
DISPOSITIF DE MISE EN CULTURE DE CELLULES, NOTAMMENT DE CELLULES HUMAINES OU ANIMALES

(43) Veröffentlichungstag der Anmeldung: 15.06.2005
(73) Patentinhaber: Pan-Biotech GmbH, 94501 Aidenbach (DE)
(72) Erfinder: SEIDL, Josef, 94474 Vilshofen a.d. Donau (DE); SCHERZE, Wilhelm, 90765 Fürth (DE)
(74) Vertreter: Benninger, Johannes
(86) Internationale Anmeldenummer: PCT/EP2002/010358
(87) Internationale Veröffentlichungsnummer: WO 2004/033616

(56) Entgegenhaltungen:
- EP-A- 0 224 800
- GB-A- 2 341 611
- US-A- 5 424 209
- US-A- 5 629 202
- US-A- 5 665 599
- US-A1- 2001 039 045
- CAMISARD V ET AL: "Inline characterization of cell concentration and cell volume in agitated bioreactors using in situ microscopy: Application to volume variation induced by osmotic stress." BIOTECHNOLOGY AND BIOENGINEERING, Bd. 78, Nr. 1, 5. April 2002 (2002-04-05), Seiten 73-80, XP002222991 ISSN: 0006-3592

## Beschreibung

Die Erfindung betrifft eine Einrichtung zur Kultivierung von Zellen verschiedenster Art, insbesondere menschlicher oder tierischer Zellen, wobei von Zellen wenigstens einer bestimmten Art jeweils eine Kultur in einer definierten Umgebung angesetzt wird und wobei die Zellen der betreffenden Kultur mit zugeordneten, flüssigen Nährmedien, Wachstumsfaktoren, Gasen oder dergleichen versorgt werden.

Kulturen der vorgenannten Art werden im allgemeinen von einzelnen Zellen angesetzt, die entweder von Gewebeteilen, von primären Kulturen, von Zell-Linien oder Zell-Stämmen durch enzymatische, mechanische oder chemische Zerteilung herrühren.

Bei bisher bekannten Einrichtungen zur Zellkultivierung werden zum Ansetzen der Kulturen in der Regel aus Kunststoff bestehende Kulturgefäße verwendet, die in CO₂-Brutschränken inkubiert werden. Diese garantieren eine konstante Temperatur (z.B. 37°C) und eine Pufferung des Mediums durch eine 5 % - 10 %ige CO₂-Begasung. Die Sauerstoffversorgung erfolgt durch einfache Diffusion. Bei den bekannten Verfahren und Einrichtungen zur Kultivierung von Zellen sind Co-Kultivierung und frei veränderliche Inkubationsbedingungen in der Regel nicht möglich.

Zur mikroskopischen Beobachtung oder zu speziellen Untersuchungen müssen die Kulturgefäße aus dem jeweiligen Brutschrank entnommen werden, wobei die Inkubation unterbrochen wird, die Zellen sich abkühlen und somit die Versuchsbedingungen nicht konstant sind.

Die bisher bekannten Verfahren und Einrichtungen zur Kultivierung von Zellen werden jedoch den Anforderungen der modernen Zellkulturtechnologie nicht mehr gerecht.

Insbesondere im Hinblick auf aktuelle Forschungsschwerpunkte in der Pharmaindustrie, die in den Bereichen Entzündung (Rheuma), Krebsbekämpfung, Herz/Kreislauf-Erkrankungen, Aids, Apoptose (programmierter Zelltod) und Blutgerinnung liegen, ist die Entwicklung und Erprobung entsprechender neuer Wirkstoffe und Medikamente mit Hilfe einer wesentlich verbesserten Einrichtung zur Kultivierung von Zellen unabdingbar, wobei eine solche Einrichtung dazu befähigt sein muß, die Substanz- und Wirkungstestung unter nahezu in-vivo-Bedingungen, d.h. mit nahezu perfekter Abbildung komplexer biologischer Systeme, vor Übertritt in die klinischen Phasen (Testung an Probanten) durchzuführen.

Mit Rücksicht auf die wie oben geschilderte Situation besteht die Forderung nach einer Möglichkeit der Simulation von Reaktionsabläufen innerhalb eines oder mehrerer Organsysteme (z.B. durch Serienschaltung von Zellkulturkammern mit Hepatozyten und anderen Zellarten, Untersuchung auf Abbauprodukte und Metabolite), damit zum einen die Zeiträume zwischen Substanzwirkungserkennung und Arzneimittelzulassung erheblich minimiert werden und zum anderen vor dem Eintritt in die klinische Testphase die notwendigen Erkenntnisse über den Wirkungsmechanismus der Substanz innerhalb eines komplexen biologischen Systems erlangt werden können.

Eine ähnliche Situation liegt beispielsweise auch im Bereich der Kosmetikindustrie vor.

Im Stand der Technik sind beispielsweise multivalente Zellkultursysteme (vgl. z.B. DE 199 15 178 A1), problemadaptierte Zellkultursysteme für spezifische Aufgabenstellungen (vgl. z.B.

WO 98/17822), Verfahren zur Replikation von Zellkulturen (vgl. z.B. WO 97/37001), oder automatisierte Zellkultursysteme (US 5 424 209) bekannt.

Ferner ist beispielsweise aus der WO 99/23206 ein Verfahren zum Mischen einer varizella-infizierten Zellkultur in Rollflaschen bekannt.

Schließlich sind aus der EP 0 999 266 A1 ein Verfahren und eine Vorrichtung zur Aufnahme einer Zellkultur bekannt, wodurch möglichst homogene Bedingungen für die molekularbiologische oder gentechnische Untersuchung von Zellen geschaffen werden sollen.

Mit Rücksicht auf die im Vorangehenden geschilderte Situation auf dem Gebiet der modernen Zellkulturtechnologie liegt der vorliegenden Erfindung nunmehr die Aufgabe zugrunde, eine neue, verbesserte Einrichtung zur Kultivierung von Zellen verschiedenster Art, insbesondere menschlicher oder tierischer Zellen zu schaffen, wobei diese Einrichtung die Nachteile bisher bekannter Systeme und Einrichtungen zur Zellkultivierung beseitigt und insbesondere die Möglichkeit bietet, hochkomplexe, biologische Vorgänge in Echtzeit und unter nahezu in-vivo-Bedingungen (d.h. wie im lebenden Organismus) bei gleichsam optimal angepaßten Lebens- und Wachstumsbedingungen der Zellen zu simulieren.

Ausgehend von einer Einrichtung zur Kultivierung von Zellen verschiedenster Art, insbesondere menschlicher oder tierischer Zellen, wobei von Zellen wenigstens einer bestimmten Art jeweils eine Kultur in einer definierten Umgebung angesetzt wird und die Zellen der betreffenden Kultur mit zugeordneten, flüssigen Nährmedien, Wachstumsfaktoren, Gasen und dergleichen versorgt werden, wird die wie vorstehend definierte Aufgabe erfindungsgemäß dadurch gelöst, daß die Einrichtung Zellkultivierungs- und Inkubationsmittel aufweist, die in der Weise ausgebildet, sind, daß es den in wenigstens einer Zellkulturkammer der Einrichtung ausgesäten Zellen ermöglicht ist, sich ihre im individuellen Falle erforderlichen Lebens- und Wachstumsbedingungen selbst einzustellen.

Die erfindungsgemäße Einrichtung weist hierbei vorzugsweise die Kombination folgender Merkmale auf:
a) Mittel zum Ingangsetzen eines Flusses frei wählbarer, definierter, flüssiger Medien in die wenigstens eine Zellkulturkammer zur kontinuierlichen Versorgung der dort ausgesäten Zellen;
b) Mittel zum Ingangsetzen eines Stromes unterschiedlicher Gase mit frei wählbaren Konzentrationen in die wenigstens eine Zellkulturkammer zur konstanten, kontinuierlichen Begasung der dort ausgesäten Zellen;
c) Mittel zum geregelten bzw. gesteuerten Beheizen der wenigstens einen Zellkulturkammer in der Art und Weise, daß hierin eine konstante Temperatur während der Dauer eines Versuches gewährleistet ist;
d) Mittel zum permanenten mikroskopischen Beobachten der innerhalb der wenigstens einen Zellkulturkammer ausgesäten Zellen, ohne während der Dauer eines Versuches Proben der Zellkultur zu entnehmen;
e) Mittel zum permanenten Messen sämtlicher relevanten Zellkulturparameter mittels entsprechender, in die wenigstens eine Zellkulturkammer integrierter Sensoren; und
f) der wenigstens einen Zellkulturkammer zugeordnete Feedback-Regelungsmittel zur Optimierung von Inkubationsbedingungen in der wenigstens einen Zellkulturkammer.

Bei den relevanten Zellkulturparametern handelt es sich insbesondere um pH-Wert, Glucose, Lactat, Sauerstoff, Elektropotential und dergleichen mehr.

Bei der erfindungsgemäßen Einrichtung ist in bevorzugter Weise eine vorgegebene Anzahl von Zellkulturkammern vorgesehen, die entweder in Reihe oder parallel geschaltet sein können, wobei innerhalb dieser vorgegebenen Anzahl von Zellkulturkammern vorzugsweise eine entsprechende Anzahl von Zellkulturen gleichzeitig angesiedelt wird.

Bei der erfindungsgemäßen Einrichtung zur Kultivierung von Zellen ist vor allem gewährleistet, daß die Zellen sämtlicher Kulturen mit flüssigen Nährmedien, Wachstumsfaktoren, Gasen oder dergleichen kontinuierlich versorgt werden, ohne daß die Zellen einer Kultur ihrer gewohnten, definierten Umgebung entnommen werden müssen, während gleichzeitig sämtliche Zellkulturen ohne Unterbrechung der Begasung permanent mikroskopisch beobachtet werden können.

Gemäß weiterer Ausgestaltung der erfindungsgemäßen Einrichtung sind Mittel vorgesehen, um während der Dauer eines Versuchs die Art der flüssigen Medien und/oder deren Strömungsrichtungen und/oder deren Verteilung und/oder deren Durchflußmengen zu variieren. Darüberhinaus können aber auch Mittel vorgesehen sein, um während der Dauer eines Versuchs die Art der Gase und/oder deren Strömungsrichtungen und/oder deren Verteilung und/oder die Begasungskonzentrationen zu variieren.

Die vorgenannten Variationsmöglichkeiten gewährleisten eine außerordentlich flexible Funktionsweise der erfindungsgemäßen Einrichtung.

Im Falle von in Reihe geschalteten Zellkulturkammern der erfindungsgemäßen Einrichtung können beispielsweise die flüssigen Medien und/oder die Gase von Zellkulturkammer zu Zellkulturkammer kontinuierlich weitergeleitet werden..

Um bei der erfindungsgemäßen Einrichtung zur Zellkultivierung während der Dauer eines Versuchs in den einzelnen Zellkulturkammern konstante Temperaturen zu gewährleisten, weist die Einrichtung in bevorzugter Weise Mittel auf, um die in den einzelnen Zellkulturen herrschenden Temperaturen permanent zu messen und als Temperatur-Istwerte einem entsprechenden Temperaturregel- bzw. Temperatursteuerkreis einzugeben, so daß die Beheizung der jeweiligen Zellkulturkammer entsprechend geregelt bzw. gesteuert wird.

Wie weiter unten im einzelnen noch näher erläutert wird, weist zu diesem Zweck jede einzelne Zellkulturkammer eine eigene Heizung auf, während oberhalb der betreffenden Zellkulturkammer jeweils ein Infrarot-Temperaturmesser angeordnet ist, der die in der betreffenden Zellkultur herrschende Temperatur mißt und diesen Temperaturmeßwert an ein Überwachungs- und Steuerungssystem meldet. Ändert sich die anfangs vorgegebene Temperatur in der wenigstens einen Zellkulturkammer, dann wird durch den Temperaturregel- bzw. Steuerkreis bewirkt, daß die Heizleistung der jeweiligen Zellkulturkammerbeheizung vermindert bzw. erhöht wird.

Die Temperaturmessung kann aber auch mit Hilfe anderer Temperatursensoren erfolgen.

Wie ebenfalls weiter unten noch näher erläutert wird, sind aus Flexibilitätsgründen die Temperaturen in den einzelnen Zellkulturkammern durch das Überwachungs- und Steuerungssystem während der gesamten Versuchsdauer frei einstellbar und veränderbar.

Eine weitere, besonders vorteilhafte Ausgestaltung der erfindungsgemäßen Einrichtung besteht darin, daß sie wenigstens eine Zellkulturkammer aufweist, in der eine gasdurchlässige Membran in der Weise angeordnet ist, daß zu beiden Seiten dieser Membran je eine Zellkultur unterschiedlicher Art zum Zwecke einer direkten Co-Kultivierung beider Zellkulturen ansetzbar ist, wobei Mittel zum Ingangsetzen eines ersten Medienflusses zu der einen Seite der Membran, d.h. der apikalen Seite mit der ersten Zellkultur, und eines gegenüber dem ersten Medienfluß unterschiedlichen, zweiten Medienflusses zu der anderen Seite der Membran, d.h. der basolateralen Seite mit der zweiten Zellkultur, vorgesehen sind.

Somit funktionieren die auf der apikalen Seite wachsenden Zellen als Deckschicht, während die Zellen auf der basolateralen Seite als Innenzellen funktionieren. Die Zellen der ersten Zellkultur und die Zellen der zweiten Zellkultur weisen hierbei durch die Membran einen recht engen Kontakt zueinander auf, so daß die Möglichkeit besteht, Austauschvorgänge innerhalb der Schichten auf der apikalen Seite und der basolateralen Seite zu untersuchen.

Darüberhinaus besteht noch die Möglichkeit, daß dann, wenn bei der erfindungsgemäßen Einrichtung gasdurchlässige Membranen mit unterschiedlichen, wählbaren Porengrößen eingesetzt werden, ein möglicher Austausch von wirksamen bioaktiven Molekülen (z.B. Wachstumsfaktoren, Hormonen, usw.) im Zuge einer derartigen Co-Kultivierung untersucht werden kann. Solche Untersuchungsmöglichkeiten sind insbesondere bei Gewebeteilen wichtig, die aus verschiedenen Zellarten aufgebaut sind, beispielsweise Übergang Endothelzellen-Fibroblasten (Adern) oder Schleimhautzellen-Fibroblasten (Darm, Magen).

Die erfindungsgemäße Einrichtung kann im übrigen mit besonderem Vorteil zur indirekten Co-Kultivierung Anwendung finden, wobei verschiedene biologische Systeme (Gewebe-/Zellarten) in entsprechenden Zellkulturkammern hintereinander geschaltet werden.

Auf diese Weise lassen sich ganze Organsysteme gleichsam nachbauen und die entsprechenden Stoffwechselvorgänge untersuchen. Diese Maßnahmen lassen sich durch ein Beispiel näher erläutern: ein an sich ungiftiger Stoff wird über den Verdauungstrakt aufgenommen und gelangt über den Blutstrom in die Leber. Die Leberzellen bauen den Stoff in Abbauprodukte um, die unter Umständen toxisch wirken können. Um dies zu überprüfen, wird die "verdächtige" Substanz in eine Inkubationskammer eingegeben, die mit Hepatozyten (Leberzellen) besiedelt ist. Über eine definierte Nährmedienversorgung (Medienfluß = "Ader") gelangen eventuell toxische Abbauprodukte in eine sich anschließende Zellkulturkammer, so daß dort z.B. aus absterbenden Nervenzellen auf eine neurotoxische Substanz geschlossen werden kann.

Gemäß einer weiteren, außerordentlich vorteilhaften Ausgestaltung der erfindungsgemäßen Einrichtung ist ein videounterstütztes mikroskopisches Beobachtungssystem zum Beobachten der wenigstens einen Zellkultur in der wenigstens einen Zellkulturkammer vorgesehen, wie dies weiter unten noch im einzelnen erläutert wird.

Schließlich besteht noch eine weitere vorteilhafte Ausgestaltung der erfindungsgemäßen Einrichtung darin, daß sie computergesteuertes Überwachungs- und Steuerungssystem aufweist, zu dem sämtliche Daten, die gewonnen werden durch
- permanentes mikroskopisches Beobachten der wenigstens einen Zellkultur innerhalb der wenigstens einen Zellkulturkammer und/oder
- permanentes Messen der relevanten Zellkulturparameter und/oder
- permanentes Messen der in der wenigstens einen Zellkultur innerhalb der wenigstens einen Zellkulturkammer herrschenden Temperatur,
zur dortigen Weiterverarbeitung und entsprechenden Beaufschlagung der Feedback-Regelungsmittel übertragbar sind.

Bei den Feedback-Regelungsmitteln handelt es sich insbesondere um Regelungsalgorithmen, die in einer Datenverarbeitungsanlage des computergesteuerten Überwachungs- und Steuerungssystems enthalten sind.

In diesem Zusammenhang ist zum permanenten Messen der relevanten Zellkulturparameter ein software-unterstütztes Meßsystem vorgesehen.

Eine kontinuierliche Messung von Zellkulturparametern läßt sich vorzugsweise durch spezielle Sonden bzw. Sensoren, beispielsweise für pH-Wert, Lactat, Elektropotential und dergleichen mehr, durchführen, wobei diese Messungen durch eine entsprechende Software ausgewertet und dargestellt werden können. Diese Art der Messung liefert gegenüber herkömmlichen Methoden exaktere Ergebnisse, wodurch bestimmte Fragestellungen analysiert werden können, die mit bisher verwendeten Meßverfahren nicht durchführbar sind.

Mit Hilfe eines bei der erfindungsgemäßen Einrichtung zum Einsatz gelangenden, software-unterstützten Meßsystems lassen sich beispielsweise bestimmte Tierversuche in der präklinischen Phase größtenteils ersetzen.

Zusammenfassend bietet die erfindungsgemäße Einrichtung zur Kultivierung von Zellen insbesondere die folgenden Vorteile:
1. Möglichkeit einer Parallelschaltung einer vorgegebenen Anzahl von Zellkulturkammern innerhalb der Einrichtung für Vergleichsmessungen.
2. Möglichkeit einer seriellen Schaltung einer vorgegebenen Anzahl von Zellkulturkammern innerhalb der Einrichtung für Organsimulation.
3. Möglichkeit einer variablen Temperaturregelung bzw. - steuerung.
4. Möglichkeit einer variablen Begasung der einzelnen Zellkulturkammern.
5. Möglichkeit einer individuellen Versorgung der Zellkulturen mit Nährsubstanzen bzw. Wirkstoffen.
6. Möglichkeit einer permanenten mikroskopischen Beobachtung des Inneren der einzelnen Zellkulturkammern und einer entsprechenden Videoaufzeichnung ohne Unterbrechung des Zellkultivierungsprozesses.
7. Möglichkeit einer permanenten Messung verschiedener Zellkulturparameter mittels integrierter Sensorik.
8. Bereitstellung eines hochwertigen Mehrwegsystems, d.h. Verarbeitung von Edelstahl und Quarzglas von voll autoklavierbarer Struktur zur Reduzierung von Abfall.

Die Erfindung wird nunmehr nachfolgend anhand von Ausführungsbeispielen näher erläutert, wobei zeigen:
Figur 1 eine schematische Ansicht einer Einrichtung zur Kultivierung von Zellen verschiedenster Art, insbesondere menschlicher oder tierischer Zellen; und
Figur 2 eine schematische Darstellung einer auf einer Basis der Einrichtung nach Figur 1 angeordneten Zellkulturkammergruppierung, zu der eine vorgegebene Anzahl von einzelnen Zellkulturkammern zusammengefaßt ist.

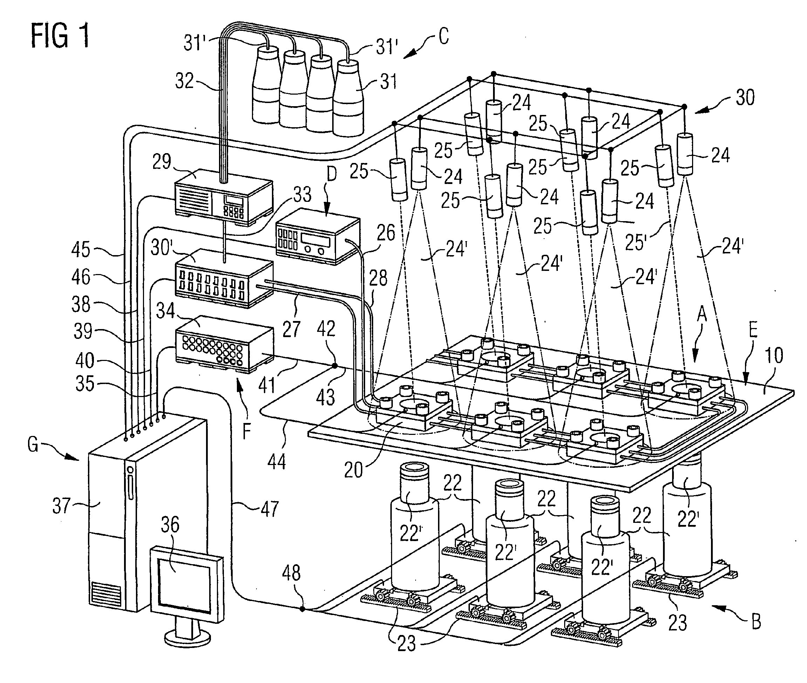

Figur 1 zeigt schematisch eine Einrichtung 30 zur Kultivierung von Zellen verschiedenster Art, wobei von Zellen wenigstens einer bestimmten Art jeweils eine Kultur in einer definierten Umgebung innerhalb einer zugeordneten Zellkulturkammer angesetzt wird und wobei die Zellen der betreffenden Kultur mit vorgewählten, flüssigen Nährmedien, Wachstumsfaktoren, Gasen und dergleichen versorgt werden.

Diese Einrichtung 30 ist insgesamt betrachtet so konzipiert, daß sie Zellkultivierungs- und Inkubationsmittel aufweist, die in der Weise ausgebildet sind, daß es den in den Zellkulturkammern der Einrichtung 30 ausgesäten Zellen ermöglicht ist, sich ihre im individuellen Falle erforderlichen Lebens- und Wachstumsbedingungen selbst einzustellen, d.h. insbesondere mit dem Ziel, daß diese Lebens- und Wachstumsbedingungen gleichsam optimiert werden.

Bei der in Figur 1 dargestellten Einrichtung 30 sind beispielsweise sechs Zellkulturkammern 20 in Form einer Zellkulturkammer gruppierung A auf einer entsprechend zugeordneten Basis 21 plaziert. Insbesondere bildet die Basis 21 ein Heizsystem E für die Inkubierung; das während der Betriebszeit die Einrichtung 30 konstante Temperaturen innerhalb jeder der Zellkulturkammern 20 gewährleistet.

Vorzugsweise erfolgt mit Hilfe dieses Heizsystems E eine elektrische Beheizung der jeweiligen Zellkulturkammer 20, wodurch eine sehr genaue Temperaturregelung ermöglicht ist. Dieses Heizsystem E ist insbesondere in der Weise ausgelegt, daß jede einzelne Zellkulturkammer 20 der Zellkulturkammergruppierung A über ihre eigene Heizung verfügt, die in der Basis 21 integriert ist.

Mit besonderem Vorteil ist das Heizsystem E mittels einer zugeordneten Software steuerbar. Zu diesem Zweck ist oberhalb der Zellkulturkammergruppierung A ein System aus Infrarot-Temperaturmessern 25 installiert, in der Art, daß jeder einzelnen Zellkulturkammer 20 ein entsprechender Infrarot-Temperaturmesser 25 zugeordnet ist. Der jeweilige Infrarot-Temperaturmesser 25 fühlt in der jeweiligen Zellkulturkammer 20 die in der Zellkultur vorherrschende Temperatur ab und meldet das entsprechende Meßergebnis permanent an ein computergesteuertes Überwachungs- und Steuerungssystem G, das im wesentlichen aus einer Datenverarbeitungsanlage 37 und einem zugehörigen Monitor 36 besteht. Die einzelnen Infrarot-Temperaturmesser 25 sind über eine gemeinsame Verbindungsleitung 45 an das Überwachungs- und Steuerungssystem G angeschlossen. Wenn sich die Anfangs vorgegebenen Temperaturen in den Zellkulturkammern 20 der Zellkulturkammergruppierung A ändern, erfolgt automatisch über das Überwachungs- und Steuerungssystem G eine Steuerung bzw. Regelung des Heizsystems E, d.h., die in der einzelnen Zellkulturkammer 20 herrschende Temperatur wird permanent auf einer konstante Temperatur eingeregelt.

Anstatt mittels Infrarot-Temperaturmessern könnte die Temperaturmessung in der einzelnen Zellkulturkammer 20 aber auch mit Hilfe anderer Temperatursensoren durchgeführt werden.

Darüberhinaus kann mit Hilfe der in dem Überwachungs- und Steuerungssystem G enthaltenen Software ermöglicht werden, daß die Temperaturen in den einzelnen Zellkulturkammern 20 der Zellkulturkammergruppierung A während der gesamten Versuchsdauer frei einstellbar und wählbar sind, falls dies aus bestimmten Gründen erforderlich sein sollte.

Zum Zwecke einer permanenten, videogestützten mikroskopischen Beobachtung des Inneren der jeweiligen Zellkulturkammer 20 ist ein Videosystem B mit einem entsprechend zugeordneten Mikroskopsystem vorgesehen. Dieses Videosystem B wird im folgenden näher erläutert.

Unterhalb jeder einzelnen Zellkulturkammer 20 der Zellkulturkammergruppierung A, die im vorliegenden Ausführungsbeispiel insgesamt sechs Zellkulturkammern aufweist, ist eine Videokamera 22 mit Mikroskopaufsatz 22' auf einem mechanisch einstellbaren Fahrtisch 23 angeordnet, somit insgesamt sechs Videokameras 22 mit zugehörigem Mikroskopaufsatz 22'. Infolgedessen beobachtet je eine Videokamera 22 mit Mikroskopaufsatz 22' je eine Zellkulturkammer 20. Nach Versuchsstart und nachdem sich aussagekräftige Bereiche in der jeweiligen in der Zellkulturkammer 20 enthaltenen Zellkultur abzeichnen, wird ein Beobachtungssektor in der Zellkulturkammer 20 festgelegt. Dieser Beobachtungssektor wird sodann durch den mechanisch einstellbaren Fahrtisch 23 mittels (nicht dargestellter) Einstellschrauben angefahren, sodann wird der Fahrtisch arretiert und das Videosystem B bleibt infolgedessen während der gesamten Versuchsdauer in der gleichen Position. Ferner wird bei Versuchsstart die Schärfe der Einstellung am jeweiligen Mikroskopaufsatz 22' einjustiert. Dieser Justiervorgang am jeweiligen Mikroskopaufsatz 22' erfolgt für sämtliche sechs Zellkulturkammern 20 und bleibt sodann unverändert bis zum Versuchsende.

Vorzugsweise wird auch das Videosystem B über die im Überwachungs- und Steuerungssystem G enthaltene Software gesteuert. Hierbei wird jede einzelne Videokamera 22 mit Mikroskopaufsatz 22' gesteuert. Dies erfolgt insbesondere in der Art, daß in frei wählbaren Zeitintervallen (beispielsweise im Minutentakt) Bilder von der jeweiligen Zellkultur in der Zellkulturkammer 20 aufgenommen werden, wobei zu dem jeweiligen Zeitpunkt einer solchen Aufnahme eine oberhalb der jeweiligen Zellkulturkammer 20 angeordnete Lichtquelle 24 die entsprechende Zellkultur beleuchtet, so daß eine ausreichende Ausleuchtung im Inneren der Zellkulturkammer 20 für die Videoaufnahmen gewährleistet ist. Wenn die Videoaufnahme beendet ist, schaltet die Steuerung die jeweilige Lichtquelle 24 aus, bis die nächste Videoaufnahme gemacht wird. Der von einer jeden Lichtquelle 24 ausgehende Lichtstrahl bzw. Lichtkegel, der in das Innere einer jeweiligen Zellkulturkammer 20 durch eine entsprechende (nicht dargestellte) Glasscheibe eintritt, ist in Figur 1 mit 24' bezeichnet.

Sämtliche Lichtquellen 24 sind über eine gemeinsame Verbindungsleitung 46 an das Überwachungs- und Steuerungssystem G angeschlossen.

Durch jeden einzelnen Lichtstrahl bzw. Lichtkegel 24 wird die jeweilige, in der Zellkulturkammer 20 enthaltene Zellkultur flächendeckend ausgeleuchtet. Es handelt sich hierbei um eine Durchleuchtungsmethode.

Anstelle einer solchen Durchleuchtungsmethode könnte aber vorgesehen sein, daß die Lichtquellen zur Ausleuchtung der in der jeweiligen Zellkulturkammer 20 enthaltenen Zellkultur unmittelbar an der jeweils zugeordneten Videokamera 22 bzw. dem jeweils zugeordneten Mikroskopaufsatz 22' angebracht sind, so daß in einem solchen Falle die Durchleuchtungsmethode durch eine Draufsichtmethode ersetzt ist.

Das Videosystem B ist ebenfalls über eine Leitung 47 an das Überwachungs- und Steuerungssystem G angeschlossen, wobei von diesem aus die Leitung 47 zu einem Knotenpunkt 48 führt, mit dem die einzelnen Videokameras 22 über entsprechend zugeordnete Leitungen verbunden sind.

Das wie oben erläuterte Videosystem B mit Mikroskopsystem stellt nur eine Ausführungsmöglichkeit dar. Eine mögliche andere Ausführungsform eines solchen Systems zur permanenten Beobachtung des Inneren der Zellkulturkammern besteht darin, daß ein einziges Beobachtungssystem, bestehend aus Videokamera und Mikroskopaufsatz, auf einem Fahrtisch installiert wird und daß dieser Fahrtisch die sechs Zellkulturkammern 20 der Zellkulturkammergruppierung A in frei wählbaren Intervallen abfährt. Die Justierung des Beobachtungssystems erfolgt für die einzelne Zellkultur bei Versuchsstart, d.h. vorzugsweise dann, nachdem sich aussagekräftige Bereiche in der jeweiligen Zellkultur abzeichnen, durch die entsprechende, im Überwachungs- und Steuerungssystem G enthaltene Software, d.h., durch das entsprechende Computerprogramm sind die sechs Anfahrpositionen des Fahrtisches, auf dem das Beobachtungssystem montiert ist, programmiert. Wegen der mechanischen Toleranzen des Fahrtisches muß jedoch ein größerer als der zu beobachtende Bereich innerhalb der einzelnen Zellkulturkammer 20 aufgenommen werden. Innerhalb dieses größeren Bereichs wird nun mittels der Software der zu beobachtende Bereich definiert. Die Software ist in der Lage, Konturen zu speichern und wiederzuerkennen, d.h., beim erneuten Anfahren einer Zellkulturkammer werden die Kultur und die Anordnung der Zellen erkannt und ein anfänglich definierter Beobachtungsbereich gespeichert.

Dieses zuletzt erläuterte Beobachtungssystem ist in den Zeichnungen im einzelnen nicht dargestellt, jedoch erfolgt die Ausleuchtung der einzelnen Zellkulturkammer 20 ebenfalls mit Hilfe der Lichtquellen 24, wie bereits weiter oben im einzelnen erläutert.

Auch in diesem Falle besteht die Möglichkeit, die Durchleuchtungsmethode durch die Draufsichtmethode zu ersetzen, d.h., die Lichtquellen zur Ausleuchtung der in der einzelnen Zellkulturkammer 20 enthaltenen Zellen können unmittelbar an der jeweils zugeordneten Videokamera 22 bzw. an dem jeweils zugeordneten Mikroskopaufsatz 22' angebracht sein.

Die in Figur 1 dargestellte Einrichtung 30 weist ferner noch ein Dosiersystem C für Flüssigkeiten (z.B. flüssige Nährmedien und dergleichen) auf, welche z.B. vier Flüssigkeitsvorratsbehälter 31 mit einer jeweils zugeordneten Flüssigkeitsentnahmeleitung 31' aufweist, wobei sodann aus diesen Flüssigkeitsentnahmeleitungen 31' ein Leitungsbündel 32 gebildet ist. Dieses Leitungsbündel 32 ist andererseits mit einem Pumpensystem 29 verbunden, durch welches die verschiedenen Zellkulturkammern 20 der Zellkulturkammergruppierung A mit frei wählbaren Flüssigkeiten, die in den Flüssigkeitsvorratsbehältern 31 enthalten sind, versorgt werden.

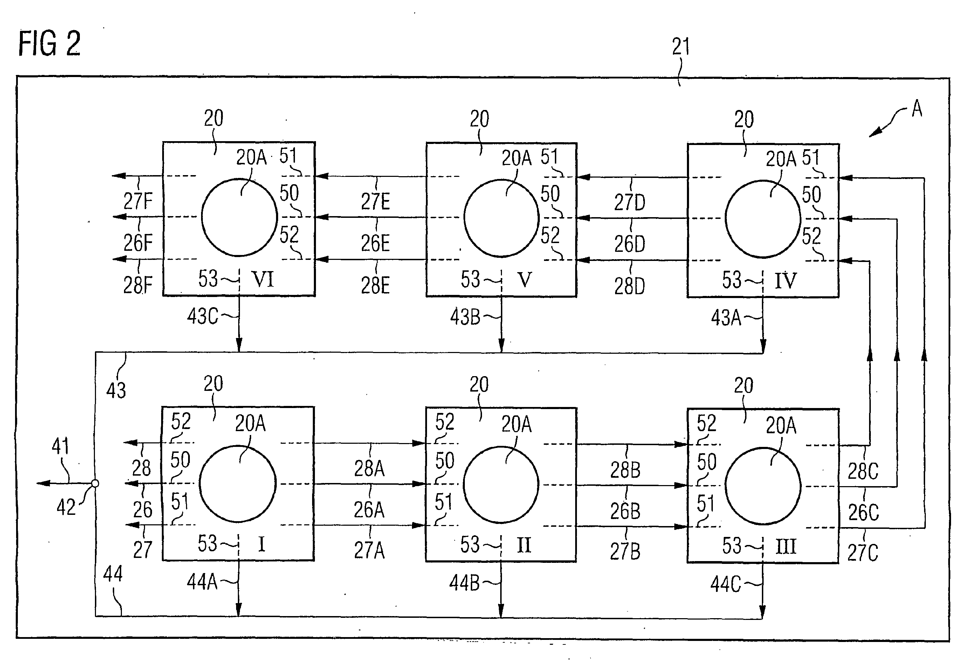

Das Pumpensystem 29 ist seinerseits über eine Leitung 33 an ein Multiventilmodul 30' angeschlossen. Die Zuführung der Flüssigkeiten zu der Zellkulturkammergruppierung A erfolgt von dem Multiventilmodul 30' aus über sterile Schlauchleitungssysteme 27 und 28, wobei diese Flüssigkeiten von den einzelnen Zellkulturkammern 20 flexibel weitergeleitet werden, d.h. von einer Zellkulturkammer zur nächsten, wie dies noch weiter unten anhand der Figur 2 näher erläutert wird.

Sowohl die Flüssigkeitszuführung als auch die Flüssigkeitsweiterleitung erfolgen über sterile Schlauchsysteme, die mit Standard-Schlauchverbinderelementen und Verteilern bei Versuchsstart installiert werden, d.h., mit entsprechenden Versorgungskanälen einer jeweiligen Zellkulturkammer 20 verbunden werden. Hierbei wird die Verbindung der Standard-Schlauchelemente (in den Zeichnungen im einzelnen nicht dargestellt) mit den zugeordneten Versorgungskanälen der jeweiligen Zellkulturkammer 20 so aufeinander abgestimmt, daß die Sterilität gewährleistet ist.

Aus Gründen der Flexibilität können die Art der Flüssigkeiten und/oder die Strömungsrichtungen und/oder die Verteilung der Flüssigkeiten und/oder deren Durchflußmengen während des Versuchs geändert bzw. gesteuert werden, wobei eine derartige Steuerung vorzugsweise durch das computergesteuerte Überwachungs- und Steuerungssystem G erfolgt. Zu diesem Zweck sind das Pumpensystem 29 mittels einer Verbindungsleitung 38 und das Multiventilmodul 30' über eine Verbindungsleitung 40 an das Überwachungs- und Steuerungssystem G angeschlossen.

Das Dosiersystem C der Einrichtung 30 erlaubt es somit, der Zellkulturkammergruppierung A unterschiedlichste Flüssigkeiten zuzuführen.

Die Einrichtung 30 weist darüberhinaus ein Begasungssystem D für unterschiedlichste Gase auf. Dieses Begasungssystem D dient dazu, die verschiedenen Zellkulturkammern 20 der Zellkulturkammergruppierung A mit unterschiedlichen Gasen, z.B. Luft, O₂, N₂, CO₂ zu begasen. Von dem Begasungssystem D aus erfolgt die Gaszuführung zu der Zellkulturkammergruppierung A mittels einer sterilen Schlauchleitung 26. Auch hierbei können die Gase von den verschiedenen Zellkulturkammern 20 unter Verwendung entsprechend zugeordneter Versorgungskanäle flexibel weitergeleitet werden, d.h., von einer Zellkulturkammer zur nächsten (vgl. Figur 2).

Gaszuführung und Gasweiterleitung erfolgen insgesamt über sterile Schläuche, die mittels Standard-Schlauchverbinderelementen und Verteilern bei Versuchsstart installiert werden. Die Verbindungen der Schlauchverbinderelemente mit den entsprechend zugeordneten Versorgungskanälen einer jeweiligen Zellkulturkammer 20 sind so aufeinander abgestimmt, daß die Sterilität gewährleistet ist. Auch bei dem Begasungssystem D können aus Flexibilitätsgründen die Art der Gase und/oder die Strömungsrichtungen und/oder die Gasverteilung und/oder die Begasungskonzentration während des Versuchs geändert bzw. gesteuert werden. Zu diesem Zweck ist wiederum das Begasungssystem D über eine Verbindungsleitung 39 an das computergesteuerte Überwachungs- und Steuerungssystem G angeschlossen, das die entsprechende Software für die Steuerung des Begasungssystems D enthält.

Schließlich weist die Einrichtung 30 zur Kultivierung von Zellen noch ein Monitoring-System F mit vorgegebenen Sensormodulen 34 auf. Mit Hilfe des Monitoring-Systems F können während der gesamten Versuchsdauer die relevanten Parameter in der jeweiligen Zellkulturkammer 20 der Zellkulturkammergruppierung A mittels entsprechend zugeordneter Sensoren gemessen, insbesondere permanent gemessen werden, wobei es sich bei diesen Parametern z.B. um pH-Wert, Glucose, Lactat, Sauerstoff, Elektropotential usw. handelt. Zu diesem Zweck steht das Monitoring-System F über eine Leitung 41, über einen Knotenpunkt 42 und von dort aus über weitere Leitungen 43 und 44 und entsprechend zugeordnete Abzweigleitungen mit den Sensoren an den einzelnen Zellkulturkammern 20 der Zellkulturkammergruppierung A der Einrichtung 30 in Verbindung.

Die von den (nicht gezeigten) Sensoren gemessenen Parameter werden von dem Monitoring-System F über eine Leitung 35 an das computergesteuerte Überwachungs- und Steuerungssystem G zur entsprechenden Weiterverarbeitung und nachfolgenden Beaufschlagung der Feedback-Regelungsmittel weitergeleitet.

Jede Zellkulturkammer 20 weist entsprechende Sensorikanschlußkanäle auf, wie dies weiter unten noch im einzelnen erläutert wird, wobei die Sensoren und die jeweils zugeordneten Kanäle so aufeinander abgestimmt sind, daß die Sterilität gewährleistet ist.

Mit besonderem Vorzug ist das Monitoring-System F in Verbindung mit dem computergesteuerten Überwachungs- und Steuerungssystem G in der Weise ausgelegt, daß das permanente Messen der relevanten Zellkulturparameter mit Hilfe eines software-unterstützten Meßverfahrens erfolgen kann (wie bereits weiter oben erläutert).

Aus Figur 2 ist die Zellkulturkammergruppierung A der Einrichtung 30 gemäß Figur 1 in schematischer Draufsicht zu ersehen. Bei der auf der Basis 21 angeordneten Zellkulturkammergruppierung A sind insgesamt sechs Zellkulturkammern 20 gleichsam in Reihe geschaltet, derart, daß sowohl die flüssigen Medien als auch die Gase von einer Zellkulturkammer 20 zur anderen, d.h. zur jeweils nachfolgend angeordneten Zellkulturkammer 20 kontinuierlich weitergeleitet werden können.

In jeder der sechs Zellkulturkammern 20 wird mindestens eine zu untersuchende Zellkultur angesiedelt, wobei jedoch im vorliegenden Ausführungsbeispiel der Einfachheit halber von sechs Zellkulturen gesprochen wird, deren jeweiligen Zellen mit definierten flüssigen Nährmedien, Wachstumsfaktoren, Gasen und dergleichen mehr zu versorgen sind.

Zu diesem Zweck wird einerseits ein Fluß frei wählbarer, definierter, flüssiger Medien und andererseits ein Strom unterschiedlicher Gase mit frei wählbaren Konzentrationen in die sechs Zellkulturkammern 20 der Zellkulturkammergruppierung A in Gang gesetzt, wobei, wie bereits weiter oben erläutert, die Zuführung der Flüssigkeiten zu der Zellkulturkammergruppierung A primär von dem Multiventilmodul 30' gemäß Figur 1 aus über die sterilen Schlauchleitungssysteme 27 und 28 erfolgt, während gleichzeitig die Gaszuführung zu der Zellkulturkammergruppierung A von dem Begasungssystem D gemäß Figur 1 aus mittels der sterilen Schlauchleitung 26 erfolgt.

Zur Erleichterung der Übersicht sind die sechs aufeinanderfolgend in Reihe geschalteten Zellkulturkammern 20 mit I, II, III, IV, V und VI gekennzeichnet.

Die Schlauchleitungssysteme 27 und 28 für Flüssigkeiten und die Schlauchleitung 26 für Gase sind unmittelbar mit der ersten Zellkulturkammer I verbunden, derart, daß die Schlauchleitung 26 unmittelbar in einen Gaskanal 50 im Inneren dieser ersten Zellkulturkammer I mündet, wohingegen das Schlauchleitungssystem 27 in einen entsprechenden Flüssigkeitskanal 51 und das Schlauchleitungssystem 28 in einen Flüssigkeitskanal 52 jeweils im Inneren dieser ersten Zellkulturkammer I einmünden. Somit wird zunächst die in der ersten Zellkulturkammer I enthaltene Zellkultur mit flüssigen Medien und Gasen versorgt, woraufhin sukzessive die nachfolgenden Zellkulturkammern II bis VI in entsprechender Weise mit flüssigen Medien und Gasen versorgt werden. Im einzelnen ist die Zellkulturkammer I über Flüssigkeits-Schlauchleitungen 27A und 28A und über eine Gas-Schlauchleitung 26A mit der zweiten Zellkulturkammer II verbunden, diese wiederum über Flüssigkeits-Schlauchleitungen 27B und 28B und eine Gas-Schlauchleitung 26B mit der dritten Zellkulturkammer III verbunden, die ihrerseits wiederum über Flüssigkeits-Schlauchleitungen 27C und 28C und eine Gas-Schlauchleitung 26C mit der vierten Zellkulturkammer IV verbunden ist, während diese wiederum über Flüssigkeits-Schlauchleitungen 27D und 28D sowie über eine Gas-Schlauchleitung 26D mit der fünften Zellkulturkammer V verbunden ist, und schließlich ist die letztere über Flüssigkeits-Schlauchleitungen 27E und 28E und über eine Gas-Schlauchleitung 26E mit der sechsten Zellkulturkammer VI verbunden.

Aufgrund dieser Hintereinanderschaltung der sechs Zellkulturkammern 20, d.h. der Kammern I bis VI, mündet jede Flüssigkeits-Schlauchleitung 28A bzw. 28B bzw. 28C bzw. 28D bzw. 28E jeweils in einen Flüssigkeitskanal 52 im Inneren jeder Zellkulturkammer, jede Flüssigkeits-Schlauchleitung 27A bzw. 27B bzw. 27C bzw. 27D bzw.. 27E mündet in einen entsprechenden Flüssigkeitskanal 51 im Inneren jeder Zellkulturkammer, wohingegen jede Gas-Schlauchleitung 26A bzw. 26B bzw. 26C bzw. 26D bzw. 26E in einen entsprechenden Gas-Kanal 50 jeder Zellkulturkammer einmündet.

Von der sechsten Zellkulturkammer VI aus gehen Flüssigkeits-Ausgangsleitungen 27F und 28F und eine Gas-Ausgangsleitung 26F ab.

Infolgedessen wird ermöglicht, daß sämtliche Zellkulturen in den sechs Zellkulturkammern I bis VI sowohl mit frei wählbaren, definierten, flüssigen Medien kontinuierlich versorgt werden als auch einer konstanten, kontinuierlichen Begasung durch das Begasungssystem D gemäß Figur 1 unterworfen werden, wie im einzelnen bereits weiter oben erläutert.

Es wird darauf hingewiesen, daß in Figur 2 lediglich eine von vielen Flußrichtungsmöglichkeiten für Flüssigkeiten und Gase dargestellt ist. Durch die oben erläuterten, flexiblen Schlauchleitungssysteme für Flüssigkeiten und Gase können auch andere Zellkulturkammer-Kombinationen als die in Figur 2 gezeigte angesteuert werden.

Von ganz besonderer Bedeutung ist noch, daß die Zellkulturkammergruppierung A insgesamt permanent an das Monitoring-System F gemäß Figur 1 angeschlossen ist, damit während der gesamten Versuchsdauer alle relevanten Parameter in der jeweiligen Zellkulturkammer 20 mittels entsprechend zugeordneter Sensoren gemessen werden können. Jede der sechs Zellkulturkammern 20 ist daher in ihrem Innern mit einem entsprechenden Kanal 53 für den Sensorikanschluß ausgerüstet. Im einzelnen ist hierbei die erste Zellkulturkammer I über eine Leitung 44A, die zweite Zellkulturkammer II über eine Leitung 44B und die dritte Zellkulturkammer III über eine Leitung 44C mit einer Leitung 44 verbunden, während die vierte Zellkulturkammer IV über eine Leitung 43A, die fünfte Zellkulturkammer V über eine Leitung 43B und die sechste Zellkulturkammer VI über eine Leitung 43C mit einer Leitung 43 verbunden ist. Die Leitungen 43 und 44 führen zu einem Knotenpunkt 42, der über eine Leitung 41 mit dem Monitoring-System F gemäß Figur 1 verbunden ist.

Mit Hilfe der im Inneren einer jeden Zellkulturkammer 20 (im vorliegenden Ausführungsbeispiel die Kammern I bis VI) angeordneten Sensoren, die hier im einzelnen nicht dargestellt sind, ist es möglich, die relevanten Parameter permanent zu messen, wobei sodann die jeweiligen Meßwerte über das Monitoring-System F an das computergesteuerte Überwachungs- und Steuerungssystem G gemäß Figur 1 zur entsprechenden Weiterverarbeitung und anschließenden Beaufschlagung der ebenfalls in dem Überwachungs- und Steuerungssystem G enthaltenen Feedback-Regelungsmittel weitergeleitet werden.

Aus Figur 2 ist noch ersichtlich, daß jede Zellkulturkammer 20 der Zellkulturkammergruppierung A an ihrer Oberseite ein rundes Fenster 20A mit Glasscheibe aufweist, durch das eine flächendeckende Ausleuchtung der in der jeweiligen Zellkulturkammer 20 enthaltenen Zellkultur ermöglicht ist, wie bereits weiter oben anhand der Figur 1 im einzelnen erläutert.

Die Zellkulturkammer als solche bildet im übrigen den Gegenstand einer deutschen Patentanmeldung der gleichen Anmelderin mit der Bezeichnung "Zellkulturkammer für ein Zellkultursystem" (amtliches Aktenzeichen ............).

Mit Hilfe der erfindungsgemäßen Einrichtung zur Kultivierung von Zellen können hochkomplexe biologische Vorgänge in Echtzeit und nahezu unter in-vivo-Bedingungen simuliert werden.

Mit ganz besonderem Vorteil kann die erfindungsgemäße Einrichtung vor allem zur Erforschung von Zellfunktionen, zur Wirksamkeitsuntersuchung von Medikamenten, zur Arzneimittelentwicklung, zur CO-Kultivierung verschiedener Zelltypen und Gewebeteile, zu organtypischen Studien, zur Beobachtung von Tumorzellen in typischer Umgebung oder toxikologischen Studien angewendet werden.

Der Vollständigkeit halber wird noch darauf hingewiesen, daß sich die erfindungsgemäße Einrichtung in der Weise abwandeln läßt, daß der oben erläuterte Feedback-Regelungsmechanismus (Regelungsmittel, Regelungsalgorithmen) nicht in Funktion gesetzt wird, was also praktisch bedeutet, daß man den Zellkultivierungsvorgang sich selbst überläßt, ohne die Inkubationsbedingungen durch den Feedback-Regelungsmechanismus zu beeinflussen.

Bei dieser Betriebsvariante der erfindungsgemäßen Einrichtung zur Kultivierung von Zellen werden die Zellkulturparameter a priori eingestellt, aber während des Zellkultivierungsvorgangs nicht verändert, obwohl sie auch bei der zuletzt erläuterten Betriebsvariante permanent gemessen werden.

## Patentansprüche

1. Einrichtung zur Kultivierung von Zellen verschiedenster Art, insbesondere menschlicher oder tierischer Zellen, wobei von Zellen wenigstens einer bestimmten Art jeweils eine Kultur in einer definierten Umgebung angesetzt wird und wobei die Zellen der betreffenden Kultur mit zugeordneten, flüssigen Nährmedien, Wachstumsfaktoren, Gasen und dergleichen versorgt werden, wobei die Einrichtung (30) Zellkultivierungs- und Inkubationsmittel aufweist, die in der Weise ausgebildet sind, dass es den in wenigstens einer Zellkulturkammer (20) der Einrichtung (30) ausgesäten Zellen ermöglicht ist, sich ihre im individuellen Fall erforderlichen Lebens- und Wachstumsbedingungen selbst einzustellen, **gekennzeichnet durch** ein videounterstütztes mikroskopisches Beobachtungssystem (B) zum Beobachten der wenigstens einen Zellkultur in der wenigstens einen Zellkulturkammer (20), wobei das videounterstützte mikroskopische Beobachtungssystem (B) eine Kamera, die mit einem Mikroskopaufsatz kombiniert ist, und einen Fahrtisch, auf dem die eine Kamera installiert ist, umfasst, wobei das Beobachtungssystem (B) **durch** eine Software gesteuert wird, auf der Anfahrpositionen gespeichert sind.

2. Einrichtung nach Anspruch 1, **dadurch gekennzeichnet, dass** auf der Software Konturen gespeichert werden und Kultur und Anordnung der Zellen beim erneuten Anfahren der Kamera wieder erkannt werden.

3. Einrichtung nach Anspruch 1 oder 2, **gekennzeichnet durch** die Kombination folgender Merkmale:
a) Mittel (C) zum Ingangsetzen eines Flusses frei wählbarer, definierter, flüssiger Medien in die wenigstens eine Zellkulturkammer (20) zur kontinuierlichen Versorgung der dort ausgesäten Zellen;
b) Mittel (D) zum Ingangsetzen eines Stromes unterschiedlicher Gase mit frei wählbaren Konzentrationen in die wenigstens eine Zellkulturkammer (20) zur konstanten, kontinuierlichen Begasung der dort ausgesäten Zellen;
c) Mittel (E) zum geregelten bzw. gesteuerten Beheizen der wenigstens einen Zellkulturkammer (20) in der Art und Weise, dass hierin eine konstante Temperatur während der Dauer eines Versuches gewährleistet ist;
d) Mittel (F) zum permanenten Messen sämtlicher relevanten Zellkulturparameter mittels entsprechender, in wenigstens eine Zellkulturkammer (20) integrierter Sensoren; und
e) der wenigstens einen Zellkulturkammer (20) zugeordnete Feedback-Regelungsmittel zur Optimierung von Inkubationsbedingungen in der Zellkulturkammer (20).

4. Einrichtung nach Anspruch 1 bis 3, **dadurch gekennzeichnet, dass** die Einrichtung (30) eine vorgegebene Anzahl von Zellkulturkammer (20) aufweist, die in Reihe geschaltet sind.

5. Einrichtung nach Anspruch 1 bis 3, **dadurch gekennzeichnet, dass** die Einrichtung (30) eine vorgegebene Anzahl von Zellkulturkammern (20) aufweist, die parallel geschaltet sind.

6. Einrichtung nach einem der Ansprüche 2 bis 5, **gekennzeichnet durch** Mittel, um während der Dauer eines Versuchs die Art der flüssigen Medien und/oder deren Strömungsrichtungen und/oder deren Verteilung und/oder deren Durchflussmengen zu variieren.

7. Einrichtung nach einem der Ansprüche 2 bis 6, **gekennzeichnet durch** Mittel, um während der Dauer eines Versuches die Art der Gase und/oder deren Strömungsrichtungen und/oder Verteilung und/oder die Begasungskonzentrationen zu variieren.

8. Einrichtung nach einem der Ansprüche 1 bis 7, **gekennzeichnet durch** Mittel (25), um die in der wenigstens einen Zellkultur innerhalb der wenigstens einen Zellkulturkammer (20) herrschende Temperatur permanent zu messen und als Temperatur-Istwert einem entsprechenden Temperaturregel- bzw. Steuerkreis einzugeben, so dass die Beheizung der Zellkulturkammer (20) entsprechend geregelt bzw. gesteuert wird.

9. Einrichtung nach einem der vorhergehenden Ansprüche, **gekennzeichnet durch** wenigstens eine Zellkulturkammer (20), in der eine gasdurchlässige Membran in der Weise angeordnet ist, dass zu beiden Seiten dieser Membran je eine Zellkultur unterschiedlicher Art zum Zwecke einer direkten Co- Kultivierung beider Zellkulturen ansetzbar ist, wobei Mittel zum Ingangsetzen eines ersten Medienflusses zu der einen Seite der Membran, d.h. der apikalen Seite mit der ersten Zellkultur, und eines gegenüber dem ersten Medienfluss unterschiedlichen, zweiten Medienflusses zu der anderen Seite der Membran, d.h. der basolateralen Seite mit der zweiten Zellkultur, vorgesehen sind.

10. Einrichtung nach einem der Ansprüche 2 bis 9, **gekennzeichnet durch** ein computergesteuertes Überwachungs- und Steuerungssystem (G), zu dem sämtliche Daten, die gewonnen werden **durch**
- permanentes mikroskopisches Beobachten der wenigstens einen Zellkultur innerhalb der wenigstens einen Zellkulturkammer (20) und/oder
- permanentes Messen der relevanten Zellkulturparameter und/oder
- permanentes Messen der in der wenigstens einen Zellkultur innerhalb der wenigstens einen Zellkulturkammer (20) herrschenden Temperatur,
zur dortigen Weiterverarbeitung und nachfolgenden entsprechenden Beaufschlagung der Feedback- Regelungsmittel übertragbar sind.

11. Einrichtung nach einem der Ansprüche 2 bis 10, **gekennzeichnet durch** ein software-unterstütztes Meßsystem zum permanenten Messen der relevanten Zellkulturparameter.

12. Einrichtung nach einem der vorhergehenden Ansprüche, **dadurch gekennzeichnet, dass** die Zellkulturkammern (20) zu einer geschlossenen Zellkammergruppierung (A) zusammengefasst sind, die auf einer Basis (21) angeordnet ist, die ein Heizsystem (E) für die Inkubierung bildet.

13. Einrichtung nach einem der vorhergehenden Ansprüche, **gekennzeichnet durch** die Anwendung der Einrichtung zur indirekten Co- Kultivierung, wobei verschiedene biologische Systeme (d.h. Gewebe-/ Zellarten) in entsprechenden Zellkulturkammern (20) hintereinandergeschaltet werden.

## Claims

1. A device for culturing cells of diverse types, particularly human or animal cells, wherein in each case a culture is prepared from cells of at least one specific type in a defined environment, and wherein the cells of the relevant culture are supplied with assigned, liquid nutrient media, growth factors, gases and the like, wherein the device (30) comprises cell culturing and incubating means which are designed to make it possible for the cells established in at least one cell culture chamber (20) of the device (30) to adapt themselves to their living and growth conditions required in each individual case, **characterized by** a video-assisted microscopic observation system (B) for observing the at least one cell culture in the at least one cell culture chamber (20), wherein the video-assisted microscopic observation system (B) comprises a camera, which is combined with a microscope attachment, and a displaceable table on which the camera is installed, wherein the observation system (B) is controlled by software, on which approach positions are stored.

2. The device according to claim 1, **characterized in that** contours are stored on the software and the culture and arrangement of the cells is recognized again when the camera next approaches.

3. The device according to claim 1 or 2, **characterized by** the combination of the following features:
a) means (C) for starting a flow of freely selectable, defined, liquid media into the at least one cell culture chamber (20) so as to provide a continuous supply to the cells established there;
b) means (D) for starting a flow of different gases with freely selectable concentrations into the at least one cell culture chamber (20) so as to provide constant, continuous gassing of the cells established there;
c) means (E) for heating the at least one cell culture chamber (20) in a regulated or controlled manner so that a constant temperature is ensured therein over the duration of an experiment;
d) means (F) for permanently measuring all the relevant cell culture parameters by means of suitable sensors integrated in the at least one cell culture chamber (20), and
e) feedback control means assigned to the at least one cell culture chamber (20) so as to optimize incubation conditions in the cell culture chamber (20).

4. The device according to claims 1 to 3, **characterized in that** the device (30) has a predefined number of cell culture chambers (20) which are connected in series.

5. The device according to claims 1 to 3, **characterized in that** the device (30) has a predefined number of cell culture chambers (20) which are connected in parallel.

6. The device according to one of claims 2 to 5, **characterized by** means for varying the type of liquid media and/or the flow directions thereof and/or the distribution thereof and/or the flow rate thereof over the duration of an experiment.

7. The device according to one of claims 2 to 6, **characterized by** means for varying the type of gases and/or the flow directions thereof and/or the distribution thereof and/or the gassing concentrations over the duration of an experiment.

8. The device according to one of claims 1 to 7, **characterized by** means (25) for permanently measuring the temperature prevailing in the at least one cell culture within the at least one cell culture chamber (20) and for inputting it as an actual temperature value into a suitable temperature regulating or control circuit, so that the heating of the cell culture chamber (20) can be suitably regulated or controlled.

9. The device according to one of the preceding claims, **characterized by** at least one cell culture chamber (20) in which a gas-permeable membrane is arranged, so that a respective cell culture of different type can be established on both sides of this membrane for the purpose of a direct co-culturing of the two cell cultures, wherein means are provided for starting a first media flow to one side of the membrane, i.e. the apical side with the first cell culture, and a second media flow different from the first media flow to the other side of the membrane, i.e. the basolateral side with the second cell culture.

10. The device according to one of claims 2 to 9, **characterized by** a computer-controlled monitoring and control system (G), to which all the data obtained by
- permanent microscopic observation of the at least one cell culture within the at least one cell culture chamber (20) and/or
- permanent measuring of the relevant cell culture parameters and/or
- permanent measuring of the temperature prevailing in the at least one cell culture within the at least one cell culture chamber (20),
can be transmitted for further processing therein and subsequent corresponding actuation of the feedback control means.

11. The device according to one of claims 2 to 10, **characterized by** a software-assisted measuring system for permanently measuring the relevant cell culture parameters.

12. The device according to one of the preceding claims, **characterized in that** the cell culture chambers (20) are combined to form a closed cell chamber group (A) which is arranged on a base (21) which forms a heating system (E) for the incubation.

13. The device according to one of the preceding claims, **characterized by** the use of the device for indirect co-culturing, wherein different biological systems (i.e. tissue/cell types) in suitable cell culture chambers (20) are connected in series.

## Revendications

1. Equipement pour la culture de cellules des types les plus variées, en particulier de cellules humaines ou animales, étant donné que, pour des cellules d'au moins un type précis, on prépare chaque fois une culture dans un environnement défini, et étant donné que les cellules de la culture concernée sont approvisionnées avec des nutritifs fluides attribués, facteurs de croissance, gaz et autres choses semblables, étant donné que l'équipement (30) présente des moyens de culture de cellules et d'incubation formés de manière à ce qu'il soit permis aux cellules semées dans au moins une chambre de culture cellulaire (20) de l'équipement (30) de s'adapter soi-même aux conditions de vie et de croissance individuellement nécessaires, **caractérisé par** un système d'observation microscopique (B) assisté par vidéo pour l'observation de la ou des cultures cellulaires dans la ou les chambres de culture cellulaire (20), étant donné que le système d'observation microscopique (B) comprend une caméra combinée à un microscope rapporté et une table de conduite sur laquelle est installée la caméra, étant donné que le système d'observation microscopique (B) est commandé par un logiciel dans lequel sont programmées les positions à parcourir.

2. Equipement selon la revendication 1, **caractérisé en ce que** le logiciel enregistre des contours, et la culture et la disposition des cellules sont de nouveau détectées lorsque la caméra recommence son parcours.

3. Equipement selon la revendication 1 ou 2, **caractérisé par** la combinaison des caractéristiques suivantes :
a) moyens (C) pour la mise en marche dans la ou les chambre(s) de culture cellulaire (20) d'un flux de fluides définis, pouvant être choisis librement, pour l'approvisionnement continu des cellules qui y sont semées ;
b) moyens (D) pour la mise en marche dans la ou les chambre(s) de culture cellulaire (20) d'un courant de gaz différents avec des concentrations pouvant être choisies librement pour assurer une alimentation en gaz constante et continue des cellules qui y sont semées ;
c) moyens (E) pour le chauffage régulé ou commandé de la ou des chambre(s) de culture cellulaire (20) de telle sorte qu'une température constante y soit garantie pendant la durée d'un essai ;
d) moyens (F) pour la mesure permanente de tous les paramètres de culture cellulaire importants au moyen de capteurs intégrés correspondants dans la ou les chambre(s) de culture cellulaire (20) ; et
e) des moyens de régulation à feed-back affectés à la ou aux chambre(s) de culture cellulaire (20) pour l'optimisation de conditions d'incubation dans la chambre de culture cellulaire (20).

4. Equipement selon les revendications 1 à 3, **caractérisé en ce que** l'équipement (30) présente un nombre prédéfini de chambres de culture cellulaire (20) qui sont montées en série.

5. Equipement selon les revendications 1 à 3, **caractérisé en ce que** l'équipement (30) présente un nombre prédéfini de chambres de culture cellulaire (20) qui sont montées en parallèle.

6. Equipement selon l'une quelconque des revendications 2 à 5, **caractérisé par** des moyens destinés à varier, pendant la durée d'un essai, les types de fluides et/ou leurs sens d'écoulement et/ou leur distribution et/ou leurs débits.

7. Equipement selon l'une quelconque des revendications 2 à 6, **caractérisé par** des moyens destinés à varier, pendant la durée d'un essai, les types de gaz et/ou leurs sens d'écoulement et/ou leur distribution et/ou les concentrations d' alimentation en gaz.

8. Equipement selon l'une quelconque des revendications 1 à 7, **caractérisé par** des moyens (25) destinés mesurer en permanence la température qui prévaut dans la ou les culture(s) cellulaire(s) à l'intérieur de la ou des chambre(s) de culture cellulaire (20) et à la programmer comme valeur effective de température dans un circuit de régulation de température ou circuit de commande correspondant, ce qui permet de réguler ou de commander en conséquence le chauffage de la chambre de culture cellulaire (20).

9. Equipement selon l'une quelconque des revendications précédentes, **caractérisé par** au moins une chambre de culture cellulaire (20) dans laquelle est disposée une membrane perméable aux gaz de manière à ce qu'on puisse préparer sur chacun des deux côtés de cette membrane une culture cellulaire de type différent afin d'assurer une co-culture directe des deux cultures cellulaires, étant donné que des moyens sont prévus pour la mise en marche d'un premier flux de fluide vers l'un des côtés de la membrane, c'est-à-dire le côté apical avec la première culture cellulaire, et d'un deuxième flux de fluide différent du premier flux de fluide vers l'autre côté de la membrane, c'est-à-dire le côté basolatéral avec la deuxième culture cellulaire.

10. Equipement selon l'une quelconque des revendications 2 à 9, **caractérisé par** un système de surveillance et de commande (G) commandé par ordinateur auquel peuvent être transmises toutes les données gagnée par
- observation microscopique permanente de la ou des culture(s) cellulaire(s) à l'intérieur de la ou des chambre(s) de culture cellulaire (20) et/ou
- mesure permanente des paramètres de culture cellulaire concernés et/ou
- mesure permanente de la température qui prévaut dans la ou les culture(s) cellulaire(s) à l'intérieur de la ou des chambre(s) de culture cellulaire (20)
pour traitement ultérieur et alimentation correspondante suivante des moyens de régulation à feed-back.

11. Equipement selon l'une quelconque des revendications 2 à 10, **caractérisé par** un système de mesure assisté par logiciel pour la mesure permanente des paramètres de culture cellulaire concernés.

12. Equipement selon l'une quelconque des revendications précédentes, **caractérisé en ce que** les chambres de culture cellulaire (20) sont regroupées en un groupe fermé de chambres cellulaires (A) qui sont disposées sur une base (21) qui forme un système de chauffage (E) pour l'incubation.

13. Equipement selon l'une quelconque des revendications précédentes, **caractérisé par** l'application de l'équipement de co-culture indirecte, étant donné que différents systèmes biologiques (c'est-à-dire types de tissus / de cellules) sont montés l'un derrière l'autre dans des chambres de culture cellulaire (20) correspondantes.
